# EUROPÄISCHE PATENTSCHRIFT

(11) **EP 2 184 081 B1**
(45) Veröffentlichungstag und Bekanntmachung des Hinweises auf die Patenterteilung: **16.01.2013**
(21) Anmeldenummer: 09172869.1
(22) Anmeldetag: 13.10.2009
(51) Int. Cl.: A61M 25/00

(54) **Katheterschaft**
Catheter shaft
Tige de cathéter

(30) Priorität: 07.11.2008 DE 102008043541
(43) Veröffentlichungstag der Anmeldung: 12.05.2010
(73) Patentinhaber: Biotronik VI Patent AG, 6341 Baar (CH)
(72) Erfinder: Gielenz, Gerhard, 8620, Wetzikon (CH); Hofmann, Eugen, 8049, Zürich (CH)
(74) Vertreter: Lindner-Vogt, Karin L.

(56) Entgegenhaltungen:
- EP-A- 0 038 937
- WO-A-91/13648
- DE-A1- 4 302 693
- DE-A1-102005 007 596
- FR-A- 1 591 251
- US-A- 5 789 047
- US-A- 5 800 522

## Beschreibung

Die vorliegende Erfindung betrifft ein Verfahren zur Herstellung eines Längenabschnittes eines Katheters, den diesen Längenabschnitt umfassenden Katheter selbst und eine Vorrichtung zur Durchführung des Herstellungsverfahrens.

Der erfindungsgemäße Katheter ist zur Sondierung sowie zur Entleerung oder Füllung von menschlichen oder tierischen Hohlorganen geeignet, sowie zur Inflation beziehungsweise Deflation von einem am Katheter befestigten Ballon, wie zum Beispiel in der vaskulären Intervention, insbesondere in der PTCA oder neurovaskulären Anwendung.

Derartige Katheter, die einen langen Schaftabschnitt mit hohlem Innenquerschnitt aufweisen, sollen bei elastischem Verhalten eine hohe Zug-, Druck- sowie Torsionsbelastung aushalten können. Des Weiteren sollen sie eine hohe Berstfestigkeit aufweisen und eine Innenmantelfläche mit geringer Rauheit zur Verbesserung des Gleitverhaltens von in den Katheterschaft eingeführten Instrumenten.

Dabei besteht insbesondere die Diskrepanz zwischen dem gewünschten geringen Außendurchmesser und der damit verbundenen geringen Wandstärke des Katheterschaftes gegenüber den geforderten mechanischen Festigkeiten. Katheterschäfte mit geringem Außendurchmesser bei geringer Wandstärke und den erforderlichen mechanischen Eigenschaften lassen sich nur mit erhöhtem Fertigungsaufwand und demzufolge hohem Kostenaufwand zur Verfügung stellen.

Bekannte Katheterkonstruktionen, wie zum Beispiel in der US 6,824,553 B1, US 6,143,013 und in der US 4,425,919 dargestellt, umfassen zur Verstärkung des Katheterschaftes Metallgeflechte, die zumindest formschlüssig an durchgehenden Rohren befestigt sind. Zur Realisierung einer glatten Außenoberfläche weisen diese Katheter außerdem eine Beschichtung, ebenfalls als durchgehendes Rohr ausgestaltet, über dem Metallgeflecht auf. Zwar haben derart hergestellte Katheterschäfte vorteilhafte mechanische Eigenschaften, jedoch sind diese Katheterschäfte in ihren Außendurchmessern sowie Wandstärken derart bemessen, dass sie für Anwendungen in sehr engen Gefäßen, wie insbesondere bei neurovaskulären Operationen, nicht verwendet werden können. Des Weiteren ist das Verfahren zur Herstellung derartiger Katheterschäfte insgesamt sehr aufwändig und damit kostenintensiv.

Ein weiteres Verfahren zur Herstellung von Kathetern beziehungsweise deren Schäften besteht in der Extrusion zumindest eines Bestandteils des Katheterschaftes in Rohrform. Es ist zum Beispiel in der EP 0 650 740 A1 offenbart, dass eine äußere Schicht in Rohrform über eine bereits in Rohrform vorliegende innere Schicht eines Katheterschaftes extrudiert wird. Das heißt, dass die extrudierte Schicht somit mit einem Hohlquerschnitt vorliegt, in dem die darunter liegende, ebenfalls in Rohrform ausgebildete Schicht, angeordnet ist. Mittels Extrusionsverfahren lassen sich derzeit in Abhängigkeit vom verwendeten Material und dem angestrebten Durchmesser des Katheterschaftes Wandstärken des Schaftes von 70-80 µm realisieren. Geringere Wandstärken sind zur Zeit deshalb nicht herstellbar, weil dann beim Extrusionsprozess der Ringspalt zwischen Düse und Dorn der Extrusionsvorrichtung derart eng bemessen wäre und dadurch der Massedruck derart hoch wäre, dass damit ein Reckungsverhältnis des Extrudats entstehen würde, welches eine zu starke Molekülorientierung in Extrudierrichtung zur Folge hätte.

Eine Alternative zur Einstellung eines zu geringen Ringspaltmaßes besteht in einer höheren Extrudiergeschwindigkeit, sodass das Röhrchen schneller aus dem Extrudierwerkzeug herausgezogen wird. Auch bei diesem Verfahren würde bei zu hoher Geschwindigkeit ein zu hohes Reckungsverhältnis vorliegen. Die stark ausgeprägte Molekülorientierung in Extrudierrichtung führt zu anisotropen mechanischen Eigenschaften des Extrudats, nämlich der Erhöhung der mechanischen Festigkeit in Längsrichtung bei gleichzeitiger Verringerung der Berstfestigkeit sowie der Torsionsfestigkeit. Im Gegensatz dazu hätten ideal isotrope Katheterschäfte gleicher Dimension eine erhöhte Berst- und Torsionsfestigkeit bei ausreichender Zug- und Druckfestigkeit.

Die starke Molekülorientierung in Extrudierrichtung wirkt sich insbesondere bei extrudierten Rohren beziehungsweise Röhrchen negativ aus in dem Fall, wenn das extrudierte Rohr in radialer Richtung wie zum Beispiel durch die Einbringung eines erhöhten Innendruckes beim Aufblasen eines an den Katheterschaft angeschlossenen Ballons belastet wird. Des Weiteren wirkt sich die zu starke Molekülorientierung negativ aus bei Schweiß- oder Beschichtungsprozessen sowie auch hinsichtlich der Alterung, da in diesen Fällen kaum mehr kontrollierbare Längsschrumpfungen des Schaftes auftreten können.

Aus diesem Grund hat der in der EP 0 650 740 A1 dargestellte Katheter zwar eine Schicht in der Rohrwandung mit relativ geringer Wandstärke, diese Schicht muss aber zur Realisierung der erforderlichen mechanischen Festigkeitswerte mit einer zweiten, inneren Schicht verbunden sein, sodass insgesamt ein zweilagiges Hohlprofil des Katheterschaftes ausgebildet ist. Dieser Aufbau aus zwei Schichten bewirkt zwangsläufig einen relativ großen Durchmesser, sodass der Katheterschaft dem Einsatz in besonders engen Gefä-βen nicht zugängig ist.

Es ist des Weiteren bekannt, Katheter beziehungsweise den Schaft eines Katheters mit einer Wickelschicht zu versehen. Es offenbart die US 2005/0059957 A1 unter anderem in Figur 17 ein in Spiralform aufgeschnittenes Rohr, welches mit einer Folie bedeckt ist. Das heißt, der oder die in Spiralform verlaufenden Schlitze im Rohr sind durch die Folie abgedeckt. In Figur 18a ist eine ähnliche Ausführung dargestellt, wobei die Folie, die das in Spiralform geschlitzte Rohr abdeckt, ebenfalls komplementär zu den Schlitzen im Rohr ausgeführte Schlitze aufweist. Die erste genannte Ausführung der US 2005/0059957 A1 weist den Nachteil auf, dass durch die zwei Schichten des Katheterschaftes (erste Schicht: geschlitztes Rohr, zweite Schicht: Folie) ein relativ großer Durchmesser des Katheterschaftes hergestellt wird, der wie bereits bemerkt den Einsatz des Katheters in sehr engen Gefäßen unmöglich macht. Die zweite der genannten Ausführungsformen hat den weiteren Nachteil, dass der Katheterschaft keine geschlossene Oberfläche aufweist, sodass in ihn eingeleitete Fluide radial aus dem Schaft austreten können, bevor sie einen zum Beispiel am Katheterschaftende befestigten Ballon befüllen können.

In der WO 91/13648 wird ein Katheter offenbart, der hergestellt wird, indem ein Band um einen Mandrel gewickelt wird.

Die US 5,789,047 beschreibt die Herstellung von mehrschichtigen Rohren, wobei die Schichten durch helikales Wickeln unter einem bevorzugten Winkel von 30° aufgebracht werden.

Der Erfindung liegt daher die Aufgabe zugrunde, ein Verfahren und eine Vorrichtung zur Herstellung eines Katheters sowie einen Katheter selbst zur Verfügung zu stellen, der bei geringem Außendurchmesser des Katheterschaftes eine geringe Wandstärke aufweist und hohe Berstfestigkeit und Zug-, Druck- sowie Torsionsfestigkeit mit glatter Innenoberfläche und ausreichender Weiterverarbeitbarkeit kombiniert. Desweiteren ist es von Vorteil, wenn der Katheter insgesamt eine konische Form aufweist, da dadurch die Einführeigenschaften verbessert werden.

Diese Aufgabe wird durch das erfindungsgemäße Verfahren zur Herstellung zumindest eines Längenabschnitts eines Katheters gemäß dem Anspruch 1 sowie durch den damit hergestellten Katheter nach Anspruch 8 gelöst. Vorteilhafte Ausgestaltungen des Verfahrens sind in den Ansprüchen 2 bis 7 und vorteilhafte Ausgestaltungen des Katheters sind in den Unteransprüchen 9 bis 12 angegeben.

Es wird erfindungsgemäß ein Verfahren zur Herstellung zumindest eines Längenabschnittes eines Katheters zur Verfügung gestellt, wobei das Verfahren das Wickeln von Folie umfasst, und erfindungsgemäß das Folienband zunächst als Band-Spirale vorliegt und anschließend entlang der Mitten-Achse der Spirale auseinander gezogen wird, so dass sich einander überlappende Windungen ergeben, deren Überlappungsbereiche anschließend stoffschlüssig miteinander verbunden werden, so dass der Längenabschnitt eine geschlossene und zumindest flüssigkeitsdichte Oberfläche aufweist,
sodass der Längenabschnitt eine geschlossene und zumindest flüssigkeitsdichte Oberfläche aufweist.

Der Längenabschnitt des Katheters ist dabei insbesondere der Schaft oder ein Bereich des Schaftes eines Katheters, der als ein Hohlzylinder bestimmter Länge ausgebildet ist, an dem gegebenenfalls ein Ballon zur Aufweitung von Gewebe befestigt werden kann oder auch ein oder mehrere Bedienelemente. Die Befestigung des Ballons am Katheterschaft lässt sich durch Schweißung realisieren. Das heißt, dass der Katheter-Längenabschnitt ausschließlich aus Folienbandwindungen bestehen kann, die untereinander verbunden sind, sodass sie eine dichte Oberfläche ausbilden. Gegebenenfalls können diese Folienbandwindungen im Nachhinein mit einer dünnen Beschichtung versehen werden. Selbst ein beschichteter Längenabschnitt ist dabei allerdings erfindungsgemäß derart konzipiert, dass die Beschichtung derart dünn im Verhältnis zu der durch die Wicklungen realisierten Rohrwand ist, dass wenigstens 70 bis 90% der mechanischen Festigkeitswerte des Längenabschnitts durch die miteinander verbundenen Windungen beziehungsweise Wicklungen des Folienbandes realisiert werden.

Somit umfasst der erfindungsgemäße Katheter-Längenabschnitt keine Metallwindungen oder Drahtgeflechte, wie sie aus dem Stand der Technik bekannt sind. Bis auf den Anschluss von weiteren Katheter-Elementen wie zum Beispiel einem Ballon oder Betätigungselementen und/ oder Nachbearbeitungen müssen somit keine zusätzlichen Verfahrensschritte zur Herstellung eines Katheters vorgenommen werden.

Die Band-Spirale des Folienbandes und das nachfolgende Auseinanderziehen desselben bewirken, dass die Seitenränder des Folienbandes eine Steigung wie ein Gewinde einer Schraube aufweisen. Diese in Gewindegangform vorliegenden Seitenränder des Folienbandes sind die Ränder des in Streifenform vorliegenden Bandes, die in Längserstreckungsrichtung des Bandes verlaufen. Dabei wird das Folienband derart gewickelt, dass ein hohler Gegenstand mit geschlossener Oberfläche und gerundetem Querschnitt entsteht, wobei die Wicklung und die stoffschlüssige Verbindung des Folienbandes derart dicht sind, dass ein Austreten von im Längenabschnitt enthaltener Flüssigkeit verhindert wird.

Mit dieser Methode lässt sich in einfacher Weise ein Längenabschnitt mit Windungen herstellen, ohne dass beim Herstellungsprozess das Folienband über einen längeren Weg in Windungen zu führen ist. Außerdem lässt sich mittels dieses Herstellungsverfahrens in einfacher Weise eine zumindest leicht konische Form des Katheter-Schaftes erzeugen. Vorzugsweise ist die Wicklung und stoffschlüssige Verbindung gasdicht ausgeführt.

Um die Band-Spirale zu erhalten kann das Folienband oder mehrere Folienbänder auf ein extrem dünnwandiges Rohr gewickelt werden, wobei die Dicke der Rohrwand deutlich dünner als die Folienbanddicke ist. Die Rohrwanddicke beträgt vorzugsweise kleiner oder gleich 50 µm. Das heißt, dass in dieser Ausgestaltung der Katheterschaft erst durch das am beziehungsweise auf dem Rohr angebrachte Folienband die gewünschten mechanischen Eigenschaften erhält, wobei auch hier wenigstens 70 bis 90% der mechanischen Festigkeitswerte des Längenabschnitts durch die miteinander verbundenen Windungen beziehungsweise Wicklungen des Folienbandes realisiert werden.

Das mit dem Folienband zu versehende Rohr ohne Folienband würde nicht den üblichen an einen Katheterschaft gestellten Anforderungen genügen.

Vorteil dieser Ausgestaltung ist, dass statt eines nach dem Wickelprozess aus dem Katheterschaft zu entfernenden Kerns ein im gewickelten Katheterschaft verbleibendes Rohr benutzt wird, welches gleichzeitig zur radialen Abdichtung des Katheterschaftes dient.

Mittels des erfindungsgemäßen Verfahrens lassen sich Schäfte von Kathetern mit außerordentlich geringen Wandstärken und demzufolge geringen Außendurchmessern herstellen, die für den Einsatz im PTCA-Bereich oder für neurovaskuläre Anwendungen geeignet sind. Zu diesem Zweck besteht das Folienband bevorzugt aus einem biokompatiblen Polymer. Mittels der Wickeltechnik lässt sich ein Katheter beziehungsweise ein Katheterschaft herstellen, der durch die Überlappungsbereiche der Windungen nebeneinander angeordnete, ringähnliche Verstärkungsbereiche aufweist, die einen erheblichen Widerstand gegen Bersten bei Erhöhung des Innendrucks im Katheterschaft bewirken. Grundsätzlich ist der Längenabschnitt beziehungsweise der Katheterschaft nur aus Folienmaterial hergestellt, wodurch die Wandstärke erheblich reduziert wird im Vergleich zu aus dem Stand der Technik bekannten Lösungen. Durch den sich über die gesamte Länge des Längenabschnitts erstreckenden Überlappungsbereich, der schraubenlinienförmig verläuft, ist ebenfalls eine Verstärkung des Katheterschaftes in Längsrichtung gegeben, sodass sich auch erhöhte Zug- und Druckfestigkeitswerte einstellen. Die Durchführung des erfindungsgemäßen Verfahrens ist unkompliziert und demzufolge kostengünstig.

Bedingt durch die geringe Wandstärke und den sich daraus ergebenden relativ großen Innendurchmesser wird ein relativ großer Inflations- beziehungsweise Deflationsquerschnitt zum Gasaustausch eines am Längenabschnitt angeschlossenen Ballons zur Aufweitung von Gewebe zur Verfügung gestellt.

Dies ermöglicht die schnelle Inflation beziehungsweise Deflation eines Ballons, sodass der Blutfluss im Gefäß nicht zu lange unterbrochen wird.

Der relativ große Innendurchmesser des hergestellten Katheterschaftes erlaubt eine bequeme Einführung eines Führungsdrahtes eines Manipulationswerkzeuges.

Vorteilhafterweise wird die stoffschlüssige Verbindung des Folienbandes durch Schweißen hergestellt. Dabei muss nicht zwingend die gesamte Breite des Überlappungsbereiches des Folienbandes verschweißt werden, sondern es reicht zur Realisierung der flüssigkeitsdichten Oberfläche aus, dass nur Teilbereiche der Breite des Überlappungsbereiches stoffschlüssig verbunden werden, allerdings muss über die gesamte Länge des Überlappungsbereiches der Stoffschluss erfolgen. Die Schweißverbindung kann beispielsweise durch Wärmeeinwirkung einer externen Wärmequelle bewirkt werden. Es wird somit in einem Sinterprozess das Verschweißen der Folienmaterialien miteinander durchgeführt. Die Wärmequelle kann ein Ofen sein, in dem sich der Längenabschnitt komplett befindet. Alternativ kann die Wärmequelle eine Infrarot-Wärmequelle sein, mittels derer auch eine punktuelle Erwärmung des Überlappungsbereiches durchgeführt werden kann. Außerdem lassen sich die Folienbänder in den Überlappungsbereichen mittels Laserstrahlen miteinander verschweißen.

Die Schweißung ist dann vorteilhaft ausgeführt, wenn sie unter Erhöhung des Drucks auf das zu verbindende Folienband erfolgt. Diese Druckerhöhung kann zum Beispiel durch einen Schrumpfschlauch realisiert werden, der nach Erkalten des herzustellenden Längenabschnitts von diesem abgeschält wird.

Dieser Schrumpfschlauch besteht dabei aus PTFE oder einem ähnlichen Material und ist hochtemperaturfest ausgeführt. Alternativ kann der Druck auch in einem druckbeaufschlagbaren Wärmeofen mittels N₂ oder einem anderen Inertgas auf das Folienband aufgebracht werden.

Alternativ zur Verschweißung des Folienbandes ist vorgesehen, dass die stoffschlüssige Verbindung des Folienbandes durch Kleben realisiert wird. Dabei ist der Klebstoff vor dem Wickeln des Folienbandes auf dieses aufzutragen, und zwar in den für die Überlappung vorgesehenen Bereichen des Folienbandes. Selbstverständlich sollte auch der Klebstoff aus einem biokompatiblen Material sein.

Das erfindungsgemäße Verfahren lässt sich in einfacher und kostengünstiger Weise dadurch ausführen, dass lediglich ein Folienband zur Herstellung des Längenabschnitts gewickelt wird, wobei dieses Folienband sich nach dem Auseinanderziehen noch selbst teilweise überlappt. Das heißt, dass die in Schraubenform verlaufenden Seitenränder des Folienbandes mit demselben Folienband überlappen. Es wird somit, abgesehen von den Überlappungsbereichen, ein Katheter-Längenabschnitt zur Verfügung gestellt, der im Wesentlichen nur eine aus einer Schicht hergestellte Rohrwand aufweist. Vorteil eines solchen Längenabschnitts ist die kostengünstige Herstellung sowie die außerordentlich geringe Wandstärke, die ein großes Innenlumen des Katheterschaftes gewährleistet. Außerdem weist ein solcher Katheter-Längenabschnitt eine erhöhte Biege-Flexibilität auf.

In einer Alternative zur Herstellung eines aus nur einem Folienband gewickelten Katheters wird das Verfahren derart durchgeführt, dass wenigstens zwei Folienbänder zur Herstellung des Längenabschnitts gewickelt werden, wobei ein jedes der Folienbänder sich selbst und/oder mindestens eines der anderen Folienbänder nach dem Auseinanderziehen teilweise überlappt. Dabei kann es vorgesehen sein, dass zum Beispiel zwei Folienbänder zueinander parallel und auch mit gleicher Steigung verlaufend gewickelt werden und verschweißt oder verklebt werden. Alternativ können die zwei Folienbänder einander kreuzend gewickelt und stoffschlüssig verbunden werden. Es können auch mehr als zwei Folienbänder zum Einsatz kommen, das heißt, dass zum Beispiel zwei Folienbänder parallel zueinander verarbeitet werden und ein drittes oder mehrere weitere Folienbänder kreuzend zu diesen gewickelt und verschweißt oder verklebt werden.

Das Verfahren kann außerdem derart ausgestaltet sein, dass die Folienbänder gleichzeitig gewickelt und gleichzeitig stoffschlüssig miteinander verbunden werden. Das heißt, dass zunächst alle Wicklungen beziehungsweise Windungen hergestellt werden und dass anschließend alle stoffschlüssigen Verbindungen gleichzeitig hergestellt werden, wie zum Beispiel in einem Sinterofen.

Alternativ dazu lassen sich die Folienbänder zeitlich nacheinander wickeln und ebenfalls zeitlich nacheinander stoffschlüssig miteinander verbinden. Dabei kann vorgesehen sein, dass wie bei der Wicklung nur eines Folienbandes ein Folienband sich selbst überlappt und gleichzeitig mit bereits gewickeltem anderen Folienband überlappt und dass in den Überlappungsbereichen die stoffschlüssige Verbindung hergestellt wird. Das heißt, dass vorzugsweise bei der Wicklung eines Bandes auf einen bereits durch Wicklung hergestellten Hohlzylinder eines anderen Bandes die Windungen des zweiten Bandes mit dem ersten, bereits gewickelten Band verbunden werden.

Die geschlossene und flüssigkeitsdichte Oberfläche des Längenabschnitts muss dabei nicht unbedingt schon durch die Wicklung und stoffschlüssige Verbindung eines ersten gewickelten Folienbandes realisiert werden, sondern kann sich auch erst durch die Wicklung und Schweißung oder Verklebung eines zweiten oder mehrerer weiterer Folienbänder auf das erste Folienband ergeben.

Eine weitere Ausgestaltung des Verfahrens liegt darin, dass mehrere Folienbänder vor dem Wickeln einander überlagert werden, sodass sie einen parallelen Verlauf aufweisen. Dabei können diese einander überlagerten Folienbänder bereits vor dem Wickeln miteinander stoffschlüssig verbunden werden und nach dem Wickeln im Überlappungsbereich miteinander verschweißt oder verklebt werden, und zwar mit den eigenen Windungen oder gegebenenfalls mit den Windungen von weiteren, bereits gewickelten Folienbänder. Somit ist zum Beispiel durch eine zweimalige Wicklung von insgesamt drei Folienbänder aufweisenden Schichten eine sechslagige Rohrwandung des Längenabschnitts erzeugbar. Vorteil der durch mehrere Folienbänder hergestellten Katheter-Längenabschnitte liegt in der erhöhten Berst- und Zug- sowie Druckfestigkeit bei nur geringfügig vergrößerter Wanddicke des Längenabschnitts. Dabei kann das Wickeln sowie auch die stoffschlüssige Verbindung der Folienbänder in jeweils einem Arbeitsgang erfolgen, sodass auch die Herstellung eines aus mehreren Folienbändern gewickelten Längenabschnittes relativ kostengünstig bleibt.

Das Verfahren kann des Weiteren dadurch vorteilhaft ausgestaltet sein, dass an der Innenseite des Längenabschnitts die Ränder der Überlappungsbereiche stoffschlüssig mit Folienband verbunden werden. Wie bereits erwähnt ist zur Realisierung einer dichten Oberfläche des Längenabschnitts die stoffschlüssige Verbindung ausreichend, wenn sie an wenigstens einer Position der Breite des Überlappungsbereiches durchgeführt wird. Um aber eine glatte innere Oberfläche der Innenwand des Katheterabschnittes für verbesserte intrinsische Gleiteigenschaften zu gewährleisten, werden die Ränder der Überlappungsbereiche in den stoffschlüssigen Verbindungsprozess einbezogen.

Im Gegensatz dazu ist es vorteilhaft, wenn an der Außenwandung des herzustellenden Längenabschnittes zumindest teilweise die Ränder der Überlappungsbereiche nicht in die stoffschlüssige Verbindung mit Folienband einbezogen werden. Dadurch, dass die Ränder nicht mit darunter liegendem Folienband verschweißt oder verklebt werden, bilden sich kleine Stufen, die eine verbesserte Griffigkeit des Längenabschnitts bewirken. Vorzugsweise sollte diese Ausgestaltung für Bereiche des Längenabschnittes angewendet werden, die als Griff oder Betätigungsende dienen sollen.

In einer besonders vorteilhaften Ausgestaltung des Verfahrens ist vorgesehen, dass das Folienband mittels Extrusion hergestellt wird und sich die in Längsrichtung verlaufenden Seitenränder des zur Verfügung gestellten Folienbandes in Extrusionsrichtung erstrecken. Bei extrudiertem Folienmaterial sind die Moleküle in Extrusionsrichtung ausgerichtet. Dadurch, dass die in Längsrichtung verlaufenden Seitenränder des Folienbandes ebenfalls in Extrusionsrichtung ausgerichtet sind, liegt somit die Molekülorientierung ebenfalls in Richtung der Längsrichtung der Seitenränder des Folienbandes vor. Das heißt, dass durch die Wicklung des Folienbandes die Molekülorientierung annähernd dem Krümmungsverlauf oder Umfangsrichtung des Hohlprofilquerschnittes des Katheter-Längenabschnittes folgt. Dies bewirkt eine wesentlich größere Belastbarkeit durch einen im Inneren des Katheterschaftes vorliegenden Innendruck, der zum Beispiel zur Befüllung eines an den Katheter angeschlossenen Ballons dient. Die Folienbänder können dabei derart hergestellt werden, dass Folien größerer Breite extrudiert werden, die anschließend derart zerschnitten werden, dass die Längsränder der erzeugten Bänder in Extrusionsrichtung verlaufen.

Alternativ kann vorgesehen sein, dass Folienbänder extrudiert werden, an denen kein Seitenbeschnitt erfolgt, sondern die sofort nach dem Extrudieren, gegebenenfalls als Endlos-Material, erfindungsgemäß gewickelt werden. Dabei lässt sich die Abkühlung des zu Extrusionszwecken erwärmten Folienbandmaterials vorteilhaft für einen Aufschrumpfprozess des Folienbandes ausnutzen.

Des Weiteren ist das erfindungsgemäße Verfahren dann vorteilhaft ausgestaltet, wenn das Folienband unter Zugbelastung gewickelt wird. Durch das elastische Verhalten des Folienbandes wird eine bessere Abdichtung an sich überlagernden Windungen des Folienbandes erreicht.

Durch das erfindungsgemäße Verfahren kann ein Längenabschnitt des Katheters beziehungsweise der Katheter-Schaft hergestellt werden. Dabei soll allerdings nicht ausgeschlossen werden, dass nach dem Wickeln und stoffschlüssigen Verbinden der Folienbandwindungen noch weitere Bearbeitungsvorgänge wie zum Beispiel Beschichten, Oberflächenbehandlung, formgebende Verfahren, Wärmebehandlung oder Bestrahlung zur Eigenschaftsveränderung des Längenabschnitts erfolgen können. Das heißt, dass zum Beispiel nach dem stoffschlüssigen Verbinden noch ein Beschichten erfolgen kann, sodass der Längenabschnitt nicht lediglich aus der Wickelschicht, sondern auch noch weitere Schichten besteht. Dabei ist allerdings die Schicht, die die mechanischen Eigenschaften bestimmt, wie zum Beispiel die Zug-, Druck-, Torsions- und Biegesteifigkeit, die Schicht oder die Schichten der Folienbandwicklungen und nicht die einer etwaigen weiteren Beschichtung.

Um das Herstellungsverfahren ökonomisch zu gestalten, lassen sich theoretisch unendlich lange Katheter-Längenabschnitte anfertigen und anschließend auf gewünschte Längen zuschneiden, wobei die dann zugeschnittenen Längenabschnitte der weiteren Verarbeitung wie Verschweißung mit einem Ballon oder Befestigung von einem Griffstücke zur Verfügung gestellt werden.

Erfindungsgemäß wird außerdem ein Katheter zur Verfügung gestellt, wobei der Katheter-Längenabschnitt dadurch hergestellt ist, dass eine Folie in Bandform bereitgestellt wird, das Folienband schraubenförmig gewickelt wird und zwar derart, dass die gewindegangförmig verlaufenden Seitenränder des Folienbandes mit Folienband überlappen, und eine stoffschlüssige Verbindung im Überlappungsbereich erzeugt wird, sodass der Längenabschnitt eine geschlossene und zumindest flüssigkeitsdichte Oberfläche aufweist.

Vorteilhafterweise ist der Katheter ein solcher, wie er durch die erfindungsgemäße Herstellung eines Längenabschnittes nach wenigstens einem der beanspruchten Herstellungsverfahren zur Verfügung gestellt wird. Der Katheter erfährt durch die Wicklung der Folienbänder eine Eigenverstärkung, die mit der durch Drahtgeflechte oder zusätzliche Schichten bei herkömmlichen Kathetern erreichten Eigenverstärkung vergleichbar ist. Gegenüber den herkömmlichen Kathetern lassen sich außerdem durch die Beeinflussung der Übergänge in den Überlappungsbereichen verbesserte intrinsische Gleiteigenschaften oder auch eine verbesserte Griffigkeit zum Beispiel an der Außenoberfläche des Katheterschaftes realisieren. Der erfindungsgemäße Katheter beziehungsweise Katheterschaft lässt sich zur Spülung von Gefäßen, bevorzugt in organischem Gewebe, sowie zur Befestigung von Spreizelementen wie zum Beispiel einem Ballon verwenden. Ein derartiger Ballon wird vorteilhafterweise an das distale Ende des Katheterschaftes zumindest flüssigkeitsdicht, bevorzugt gasdicht, angeschweißt. Der Katheter beziehungsweise Katheterschaft bildet somit eine Fluidleitung zur Inflation und Deflation des daran angeschlossenen Ballons. Der erfindungsgemäße Katheter weist herstellungsbedingt eine nur geringe Wandstärke auf, die es ermöglicht, einen relativ großen Innendurchmesser bei vergleichbar geringem Außendurchmesser zu realisieren. Das heißt, dass der erfindungsgemäße Katheter leichter in und durch Gefäße ein- beziehungsweise durchführbar ist und gleichzeitig aufgrund seines geringen Querschnitts eine nur geringe Blockadewirkung im Gefäß hat. Durch die verbesserten intrinsischen Gleiteigenschaften lässt sich ein Werkzeug im Inneren des Katheters beziehungsweise Katheterschafts leicht ein- und hindurchführen. Dadurch, dass der Katheterschaft lediglich gewickelt und stoffschlüssig verbunden ist, ist seine Herstellung einfach und unkompliziert, sodass die Herstellung kostengünstig realisierbar ist.

Der Katheter ist dadurch vorteilhaft ausgestaltet, dass er am Schaft Bereiche mit unterschiedlichen Durchmessern aufweist. Dabei ist insbesondere die konische Form günstig, wobei das Ende der Konusform mit dem geringeren Durchmesser bevorzugt das distale Ende des Katheterschafts sein sollte. Bei Vorliegen einer Konusform beziehungsweise einer Hohlkegelabschnittsform liegen die Windungen der Folienbänder somit als räumliche Spiralen vor. Die unterschiedlichen Durchmesserbereiche des Katheterschaftes beziehungsweise seines Längenabschnittes können durch die Steigung der Wicklung und den Überlappungsgrad beeinflusst werden. Die Bereiche des Längenabschnittes des Katheters, die einen geringeren Durchmesser aufweisen, weisen demzufolge auch ein geringeres axiales Widerstandsmoment auf. Das axiale Widerstandsmoment kann allerdings wiederum dadurch erhöht werden, dass über eine bestimmte Länge mehr Wicklungen vorhanden sind und somit in diesem Bereich die Wandstärke etwas erhöht wird. Die Bereiche mit verringertem Durchmesser lassen sich insbesondere vorteilhaft zur Einführung in engere Gefäße verwenden.

Eine besondere Ausgestaltung des erfindungsgemäßen Katheters liegt darin, dass zumindest ein Teilbereich des Katheterschaftes einen elliptischen Hohlquerschnitt aufweist. Bereiche des Katheterschaftes mit elliptischem Querschnitt lassen sich vorteilhafterweise zur Positionierung in Gefäßen verwenden, in denen eine breitere und flachere Ausgestaltung des Katheters vorteilhaft ist.

Zur Realisierung des geringen Außendurchmessers und der geringen Wandstärke des Katheters beziehungsweise Katheterschaftes ist vorgesehen, dass die Folienbanddicke geringer ist als 80 µm. Damit sind wesentlich dünnere Katheter als bisher bekannt produzierbar. Es sind somit Katheter mit Durchmessern von 0,5-1 mm herstellbar, welche sich insbesondere für die PTCA- oder neurovaskuläre Anwendung eignen.

Bei der Herstellung eines Katheters, welcher aus wenigstens zwei Folienbändern gewickelt ist, kann jedes der verwendeten Folienbänder aus einem jeweils unterschiedlichen Material bestehen. Es bietet sich dabei an, dass das die Innenwand herstellende Folienband auf Grund geringer Rauhigkeitswerte gute Gleiteigenschaften aufweist und das die Außenoberfläche ausbildende Folienband eine gute Verschweißbarkeit mit zum Beispiel einem Ballon aufweist. Wie bereits zum Herstellungsverfahren erwähnt, kann vorgesehen sein, dass ein Folienband aus zwei Lagen zur Verfügung gestellt wird, was anschließend zum Katheterschaft gewickelt wird. Dabei können auch die beiden Lagen des zu wickelnden Folienbandes aus unterschiedlichen Materialien bestehen. Die verwendeten unterschiedlichen Materialien können untereinander hinsichtlich ihrer Verschweißbarkeit im Wesentlichen inkompatibel sein wie zum Beispiel Polyamid und Polyimid. Um dennoch eine stoffschlüssige Verbindung mittels Verschweißung der Folienbänder realisieren zu können, lassen sich zum Beispiel Polyimid polyamid-typische Bestandteile beimischen.

Grundsätzlich ist vorgesehen, dass das Folienband aus einem biokompatiblen Polymer besteht. Wenigstens eines der verwendeten Folienbänder sollte mit einem Nylon-Ballon verschweißbar sein. Des Weiteren bietet es sich an, Folienbänder mit Fluorkunststoff oder einer Textur zur Realisierung von guten Gleiteigenschaften an der Innenoberfläche des Katheterschaftes zu verwenden.

Vorteilhafterweise sind die verwendeten Polymere zur Herstellung des Folienbandes PEEK, PI, PS, PES und/oder PA 12, welches insbesondere für die Anbindung von konventionellen PA 12-Ballonen geeignet ist.

Vorteilhafterweise hat der erfindungsgemäß hergestellte Längenabschnitt des Katheters eine Hohlzylinderform oder eine Hohlkegelabschnittsform. Zur Herstellung einer Hohlkegelabschnittsform lassen sich Folienbänder mit nichtkonstanter Breite einsetzen, die, gegebenenfalls unter Änderung der Steigung, gewickelt werden. Somit lässt sich eine unterschiedliche Biegesteifigkeit in unterschiedlichen Abschnitten realisieren.

Außerdem lassen sich Bombierungen in Abschnitten des Katheterschaftes erzeugen, sodass in diesen Abschnitten der Katheterschaft ein erhöhtes axiales Widerstandsmoment aufweist. Diese hinsichtlich der Biegesteifigkeit verbesserten Bereiche lassen sich insbesondere in den Abschnitten des Katheterschaftes einsetzen, in denen bei Anwendung mit einer verstärkten Biegebelastung zu rechnen ist.

Die Erfindung wird anhand der beiliegenden Zeichnungen beschrieben. Es zeigt dabei
- Figur 1:: einen erfindungsgemäß hergestellten Längenabschnitt des Katheters in Hohlzylinderform,
- Figur 2:: einen erfindungsgemäß hergestellten Längenabschnitt eines Katheters mit konusförmigem Bereich,
- Figur 3:: den in Figur 2 dargestellten konusförmigen Längenabschnitt mit Darstellung der Windungsverläufe aller verarbeiteten Folienbänder.

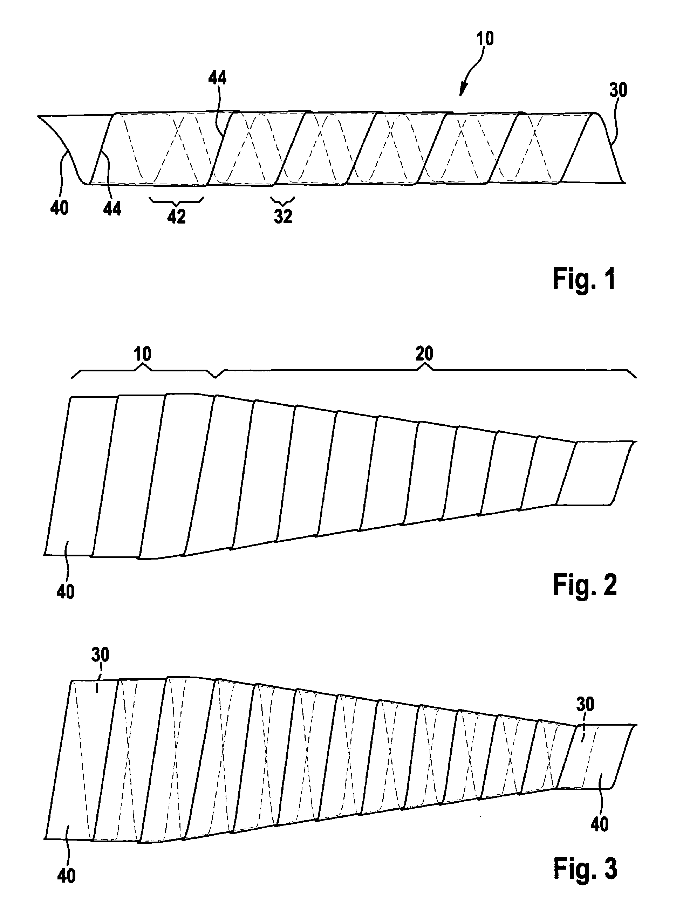

Der in Figur 1 dargestellte Längenabschnitt des Katheterschaftes ist ein hohlzylinderförmiger Bereich 10 mit unbeschnittenen Enden. Das heißt, dass zur einfacheren Anbindung mittels Schweißen oder Kleben von einem Ballon oder einem Griffstück die in Figur 1 dargestellten Enden des Längenabschnitts beschnitten werden sollten. Der in Figur 1 dargestellte Längenabschnitt ist aus zwei Folienbändern 30 und 40 gewickelt, wobei das zweite Folienband 40 über die Wicklungen des ersten Folienbandes 30 gewickelt ist. Es ist ersichtlich, dass die beiden Folienbänder 30 und 40 entgegen gesetzte Steigungen aufweist. Das heißt, dass das erste Folienband 30 eine rechtsgängige Steigung und das zweite Folienband 40 eine linksgängige Steigung aufweisen. Das führt dazu, dass sich die beiden Folienbänder 30 und 40 kreuzend überlappen. Eine derartige kreuzende Überlappung hat den Vorteil der gleichmäßigen Verteilung der Überlappungsbereiche über die Mantelfläche des Längenabschnitts beziehungsweise Katheterschaftes. Es ist somit eine gleichmäßige Torsionssteifigkeit bei Einleitung eines links- oder auch rechtsdrehenden Torsionsmomentes gewährleistet. Das gestrichelt dargestellte erste Folienband 30 ist dabei derart gewickelt, dass sich seine Windungen zumindest teilweise in einem Überlappungsbereich 32 überdecken. Das heißt, dass bei Verschweißung der Überlappungsbereiche 32 des ersten Folienbandes 30 oder Verklebung dieser Überlappungsbereiche bereits ein funktionsfähiger Katheterschaft herstellbar wäre. Wie in Figur 1 dargestellt sind allerdings zur Querschnittsverstärkung über die Windungen des ersten Folienbandes 30 noch zusätzlich die Windungen des zweiten Folienbandes 40 gelegt, wobei dieses zweite Folienband 40 ebenfalls durch eigene Windungen Überlappungsbereiche 42 realisiert. Die stoffschlüssige Verbindung der Folienbänder kann somit die Verbindung des ersten Folienbandes 30 in seinem Überlappungsbereich 32 mit sich selbst umfassen, sowie auch die stoffschlüssige Verbindung in den Überlappungsbereichen 42 des zweiten Folienbandes 40. Dadurch würde allerdings ein Längenabschnitt eines Katheters mit im Wesentlichen zwei voneinander unabhängig ausgeführten und ineinander angeordneten Rohren hergestellt werden, die theoretisch Relativbewegungen zueinander ausführen könnten. Um dies zu vermeiden und um die Festigkeit des Katheterschaftes zu erhöhen kann demzufolge vorgesehen sein, dass die stoffschlüssige Verbindung nicht nur in den Überlappungsbereichen 32 und 42 der einzelnen Folienbänder untereinander erfolgt, sondern dass die stoffschlüssige Verbindung auch die Verbindung des ersten Folienbandes 30 mit dem zweiten Folienband 40 umfasst.

Die in Figur 1 dargestellten Seitenränder 44 des zweiten Folienbandes 40 verlaufen parallel zueinander, sodass bei gleichbleibender Steigung der Wicklung ein konstant breiter Überlappungsbereich 42 ausgebildet wird.

Abweichend von der in Figur 1 dargestellten Ausführungsform ist es auch möglich, dass, wie bereits erwähnt, der Längenabschnitt nur durch die Windungen eines Folienbandes hergestellt wird, oder dass bei Verwendung von mehreren Folienbändern die übereinander gewundenen Folienbänder die gleiche Steigungsrichtung aufweisen.

In Figur 2 ist eine besondere Ausführungsform eines erfindungsgemäßen Katheters beziehungsweise Katheterschaftes dargestellt, welcher einen hohlzylinderförmigen Bereich 10 und einen hohlkegelabschnittsförmigen Bereich 20 aufweist. Das heißt, dass in dem hohlkegelabschnittsförmigen Bereich 20 der Katheterschaft Bereiche mit unterschiedlichen Durchmessern aufweist.

Wie in Figur 3 dargestellt, lässt sich auch ein derart konisch verlaufender Längenabschnitt durch Wicklung von einem Folienband oder auch zwei Folienbändern 30 und 40 realisieren. Dabei sind in ähnlicher Weise wie in Figur 1 dargestellt die beiden Folienbänder 30 und 40 einander kreuzend gewickelt. Zur Realisierung der Konusform bietet sich an, dass das Folienband zunächst in Spiralform vorliegt und anschließend zu einer dreidimensionalen, konischen Spiralform auseinander gezogen wird.

### Bezugszeichenliste

- Hohlzylinderförmiger Bereich: 10
- Hohlkegelabschnittsförmiger Bereich: 20
- Erstes Folienband: 30
- Überlappungsbereich des ersten Folienbandes: 32
- Zweites Folienband: 40
- Überlappungsbereich des zweiten Folienbandes: 42
- Seitenrand: 44

## Patentansprüche

1. Verfahren zur Herstellung zumindest eines Längenabschnittes eines Katheters, wobei das Verfahren das Wickeln von in Bandform bereitgestellter Folie umfasst, **dadurch gekennzeichnet, dass**
- das Folienband zunächst als Band-Spirale vorliegt und anschließend entlang der Mitten-Achse der Spirale auseinander gezogen wird, so dass sich einander überlappende Windungen ergeben, deren Überlappungsbereiche anschließend stoffschlüssig miteinander verbunden werden,
so dass der Längenabschnitt eine geschlossene und zumindest flüssigkeitsdichte Oberfläche aufweist.

2. Verfahren zur Herstellung eines Längenabschnittes eines Katheters nach Anspruch 1, **dadurch gekennzeichnet, dass** die stoffschlüssige Verbindung durch Schweißen realisiert wird.

3. Verfahren zur Herstellung zumindest eines Längenabschnittes eines Katheters nach Anspruch 2, **dadurch gekennzeichnet, dass** die Schweißung unter Erhöhung des Drucks auf das zu verbindende Folienband erfolgt.

4. Verfahren zur Herstellung eines Längenabschnittes eines Katheters nach Anspruch 1, **dadurch gekennzeichnet, dass** die stoffschlüssige Verbindung durch Kleben realisiert wird.

5. Verfahren zur Herstellung eines Längenabschnittes eines Katheters nach wenigstens einem der vorhergehenden Ansprüche, **dadurch gekennzeichnet, dass** lediglich ein Folienband zur Herstellung des Längenabschnittes gewickelt wird, wobei dieses Folienband sich selbst teilweise überlappt.

6. Verfahren zur Herstellung eines Längenabschnittes eines Katheters nach wenigstens einem der Ansprüche 1 bis 4, **dadurch gekennzeichnet, dass** wenigstens zwei Folienbänder zur Herstellung des Längenabschnittes gewickelt werden, wobei ein jedes der Folienbänder sich selbst und/oder mindestens eines der anderen Folienbänder teilweise überlappt.

7. Verfahren zur Herstellung eines Längenabschnittes eines Katheters nach wenigstens einem der vorhergehenden Ansprüche, **dadurch gekennzeichnet, dass** das Folienband mittels Extrusion hergestellt wird und die in Längsrichtung verlaufenden Seitenränder des zur Verfügung gestellten Folienbandes in Extrusionsrichtung ausgerichtet sind.

8. Katheter, bei dem wenigstens ein Längenabschnitt zumindest teilweise durch Wickeln von Folie hergestellt ist, **dadurch gekennzeichnet, dass** der Längenabschnitt nach dem Verfahren nach mindestens einem der Ansprüche 1 bis 7 hergestellt ist.

9. Katheter nach Anspruch 8, **dadurch gekennzeichnet, dass** der Längenabschnitt Bereiche mit unterschiedlichen Durchmessern aufweist.

10. Katheter nach wenigstens einem der Ansprüche 8 oder 9, **dadurch gekennzeichnet, dass** die Folienbanddicke geringer ist als 80 µm.

11. Katheter nach wenigstens einem der Ansprüche 8 bis 10, **dadurch gekennzeichnet, dass** der Längenabschnitt aus wenigstens zwei Folienbändern aus jeweils unterschiedlichem Material gewickelt ist.

12. Katheter nach wenigstens einem der Ansprüche 8 bis 11, **dadurch gekennzeichnet, dass** der Längenabschnitt eine Hohlzylinderform oder eine Hohlkegelabschnittsform aufweist.

## Claims

1. A method for producing at least one longitudinal portion of a catheter, wherein the method comprises the winding of film provided in tape form, **characterised in that**
- the film tape is initially provided as a tape spiral and is then drawn apart along the central axis of the spiral such that overlapping windings are produced, of which the overlapping regions are then integrally bonded to one another so that the longitudinal portion has a closed and at least liquid-tight surface.

2. The method for producing a longitudinal portion of a catheter according to Claim 1, **characterised in that** the integral bond is produced by means of welding.

3. The method for producing a longitudinal portion of a catheter according to Claim 2, **characterised in that** the welding is carried out with an increase in the pressure on the film tape to be connected.

4. The method for producing a longitudinal portion of a catheter according to Claim 1, **characterised in that** the integral bond is produced by gluing.

5. The method for producing a longitudinal portion of a catheter according to at least one of the preceding claims, **characterised in that** only one film tape is wound to produce the longitudinal portion, wherein said film tape partially overlaps itself.

6. The method for producing a longitudinal portion of a catheter according to at least one of Claims 1 to 4, **characterised in that** at least two film tapes are wound to produce the longitudinal portion, wherein each of the film tapes partially overlaps itself and/or at least one of the other film tapes.

7. The method for producing a longitudinal portion of a catheter according to at least one of the preceding claims, **characterised in that** the film tape is produced by means of extrusion, and the side edges of the film tape provided, extending in longitudinal direction, are aligned in the extrusion direction.

8. A catheter, wherein at least one longitudinal portion is produced at least partially by winding film, **characterised in that** the longitudinal portion is produced by the method according to at least one of Claims 1 to 7.

9. The catheter according to Claim 8, **characterised in that** the longitudinal portion has regions of different diameters.

10. The catheter according to at least one of Claims 8 or 9, **characterised in that** the film tape thickness is less than 80 µm.

11. The catheter according to at least one of Claims 8 to 10, **characterised in that** the longitudinal portion is wound from at least two film tapes made of different material.

12. The catheter according to at least one of Claims 8 to 11, **characterised in that** the longitudinal portion has a hollow cylindrical form or a hollow conical portion form.

## Revendications

1. Procédé pour la fabrication d' au moins une portion de longueur d'un cathéter, le procédé comprenant l'enroulement d'un film disponible sous forme de bande, **caractérisé en ce que**
- la bande de film est tout d'abord disponible sous forme de spirale de bande, puis elle est séparée le long de l'axe médian de la spirale, de manière à former des spires superposées les unes par rapport aux autres, dont les zones de superposition sont ensuite reliées entre elles par liaison de matière,
de manière à ce que la portion de longueur présente une surface fermée et au moins étanche au fluide.

2. Procédé pour la fabrication d'une portion de longueur d'un cathéter selon la revendication 1, **caractérisé en ce que** l'assemblage par liaison de matière est réalisé par une soudure.

3. Procédé pour la fabrication d'une portion de longueur d'un cathéter selon la revendication 2, **caractérisé en ce que** la soudure est réalisée en augmentant la pression sur la bande de film à relier.

4. Procédé pour la fabrication d'une portion de longueur d'un cathéter selon la revendication 1, **caractérisé en ce que** l'assemblage par liaison de matière est réalisé par un collage.

5. Procédé pour la fabrication d'une portion de longueur d'un cathéter selon l'une au moins des revendications précédentes, **caractérisé en ce qu'**une seule bande de film est enroulée pour la fabrication de la portion de longueur, dans lequel cette bande de film se chevauche elle-même par endroits.

6. Procédé pour la fabrication d'une portion de longueur d'un cathéter selon l'une au moins des revendications 1 à 4, **caractérisé en ce qu'**au moins deux bandes de film sont enroulées pour la fabrication de la portion de longueur, dans lequel chacune des bandes de film se chevauche elle-même et/ou chevauche au moins l'une des autres bandes de film par endroits.

7. Procédé pour la fabrication d'une portion de longueur d'un cathéter selon l'une au moins des revendications précédentes, **caractérisé en ce que** la bande de film est réalisée par extrusion, et les bords latéraux s'étendant dans le sens longitudinal de la bande de film disponible sont orientés dans la direction d'extrusion.

8. Cathéter, dans lequel au moins une portion de longueur est fabriquée au moins partiellement par l'enroulement d'un film, **caractérisé en ce que** la portion de longueur est réalisée à l'aide du procédé selon l'une des revendications 1 à 7.

9. Cathéter selon la revendication 8, **caractérisé en ce que** la portion de longueur comporte des régions avec des diamètres différents.

10. Cathéter selon l'une au moins des revendications 8 ou 9, **caractérisé en ce que** l'épaisseur de la bande de film est inférieure à 80 µm.

11. Cathéter selon l'une au moins des revendications 8 à 10, **caractérisé en ce que** la portion de longueur est enroulée à partir d' au moins deux bandes de film, constituées chacune d'un matériau différent.

12. Cathéter selon l'une au moins des revendications 8 à 11, **caractérisé en ce que** la portion de longueur présente une forme de cylindre creux, ou une forme d'onglet de cône creux.
